# EUROPEAN PATENT APPLICATION

(11) **EP 2 113 281 A1**
(43) Date of publication of application: **04.11.2009**
(21) Application number: 09158865.7
(22) Date of filing: 27.04.2009
(51) Int. Cl.: A61M 31/00, A61M 25/00, A61M 5/142

(54) **Transvenous soaker catheter**

(30) Priority: 01.05.2008 US 113742
(71) Applicant: Curtis, Guy P., San Diego, CA 92110 (US)
(72) Inventor: Curtis, Guy P., San Diego, CA 92110 (US)
(74) Representative: Gates, Marie Christina Esther

(57) **Abstract**

A system and method for infusing a fluid medicament requires an indwelling catheter having an infusion member affixed to its distal end. A source of the fluid medicament is connected in fluid communication with the catheter's proximal end. In use, the catheter is positioned in the venous system of a patient with the infusion member embedded and stabilized in a volume of tissue. A pump is then activated to infuse fluid medicament from the source into extracellular fluid in the volume of tissue, at a predetermined fluid flow rate and for a predetermined time duration.

## Description

### FIELD OF THE INVENTION

The present invention pertains generally to indwelling infusion catheters. More particularly, the present invention pertains to catheters that can be maneuvered to a pre-selected site in the venous system where an infusion member, at the distal end of the catheter, can be embedded into a target volume of tissue. The present invention is particularly, but not exclusively, useful as an indwelling catheter for infusing a fluid medicament into extracellular fluid in the tissue volume at a predetermined fluid flow rate and for a specified time duration.

### BACKGROUND OF THE INVENTION

Heretofore, when organs inside the body have required therapeutic treatment, the typical practice has been to orally or intravenously administer medicaments. Typically, these medicaments (i.e. drugs) transit to and from the target organ through the body's blood stream. In many instances, however, this unnecessarily exposes the entire body to the effect. And, it can be quite problematic due to the fact an efficacious concentration of the fluid medicament in one area of the body can be toxic in others.

Not infrequently, it happens that only a portion of an organ's tissue requires therapeutic treatment. Moreover, it may be desirable, and indeed necessary, to treat this portion of organ tissue with relatively high concentrations of a fluid medicament for an extended period of time. An effective way to do this is by infusing a fluid medicament directly into extracellular fluid in the target tissue. Fortunately, with such infusions the effect can remain somewhat localized as the medicament diffuses down its concentration gradient from the site of application to more distant sites in the tissue. Further, the effect may even be limited to only the particular organ and, thus, effectively isolated from the blood stream. Nevertheless, under such circumstances it is necessary that the administration of the fluid medicament be properly controlled.

Various types of catheter systems are commercially available for use in the vasculature of a patient. Typically, each catheter system is specially designed to perform a particular function, or functions, for a specific application. Of particular interest for the present invention are indwelling catheters that can remain in situ in the venous system of a patient's vasculature for extended periods of time.

In light of the above, it is an object of the present invention to provide a transvenous soaker catheter, and its method of use, that is able to selectively deliver substances (i.e. fluid medicaments) at adjustable concentrations into the extracellular fluid, at a particular location in a target organ. Another object of the present invention is to provide a transvenous soaker catheter that effectively limits the exposure of an administered fluid medicament to targeted organ tissue. Still another object of the present invention is to provide a transvenous soaker catheter, and its method of use, that minimizes system toxicity while allowing the local administration of much higher concentrations of a drug (i.e. fluid medicament) in a predetermined area. Yet another object of the present invention is to provide a transvenous soaker catheter, and its method of use, that is easy to use, is relatively simple to manufacture, and is comparatively cost effective.

### SUMMARY OF THE INVENTION

In accordance with the present invention, a system for infusing a fluid medicament into a volume of tissue includes a catheter having a proximal end and a distal end. A source of the fluid medicament is attached in fluid communication with the proximal end of the catheter, and a controller is provided to establish a predetermined fluid flow rate for the flow of the fluid medicament from the source to the catheter. Also, the controller includes a timer that allows control over the time duration of the fluid medicament flow from the source to the catheter.

An infusion member is affixed to the distal end of the catheter. Preferably, the infusion member is an elongated needle like structure that extends in a distal direction from the distal end of the catheter. For one embodiment of the infusion member, it is formed with at least one laser pin hole. In another embodiment, at least one biodegradable fiber extends from the infusion member. For either embodiment, after the fluid medicament has been pumped through the catheter, the fluid medicament leaves the infusion member though the pin hole(s) or the biodegradable fiber(s).

As envisioned for the present invention, the infusion member can be associated with a stabilizing element at the distal end of the catheter. For example, a helical wire (i.e. cork-screw shaped wire) can be attached to the distal end of the catheter to surround the infusion member. As another example, the infusion member itself can be helical shaped.

In the operation of the present invention, the intravenous soaker catheter is maneuvered through the venous system until the infusion member is positioned at an intended target site. The infusion member is then embedded into tissue at the target site, and stabilized. In many instances, the length of the infusion member will be sufficient to stabilize it at the target site. On the other hand, as indicated above, stabilization can also be accomplished by screwing a helical stabilization wire that is mounted on the catheter, into tissue at the target site. The infusion member itself may also be helical shaped and, thus, it can be similarly screwed into tissue at the target site for stabilization.

Once the catheter has been properly positioned, and the infusion member properly stabilized, the controller can be activated. Specifically, in accordance with instructions from the controller, the fluid pump can be operated to infuse fluid medicament from the fluid source into extracellular fluid in the target volume of fluid. This can be done at a predetermined fluid flow rate, for a predetermined time duration. As envisioned for the present invention, the infusion of fluid medicament can be continuously, or periodically, accomplished over extended periods of time (e.g. greater than five minutes).

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Fig. 1 is a schematic view of a system for an indwelling, transvenous soaker catheter in accordance with the present invention;
Fig. 2 is a cross section view of tissue adjacent a vein in the venous system with a soaker catheter of the present invention operationally embedded into the tissue;
Fig. 3 is an alternate embodiment of the infusion member for a soaker catheter in accordance with the present invention;
Fig. 4 is a view of an infusion member with a helical screw for stabilizing the infusion member in the venous system; and
Fig. 5 is another alternate embodiment showing the infusion member formed as a helical screw.

### DESCRIPTION OF THE PREFERRED EMBODIMENTS

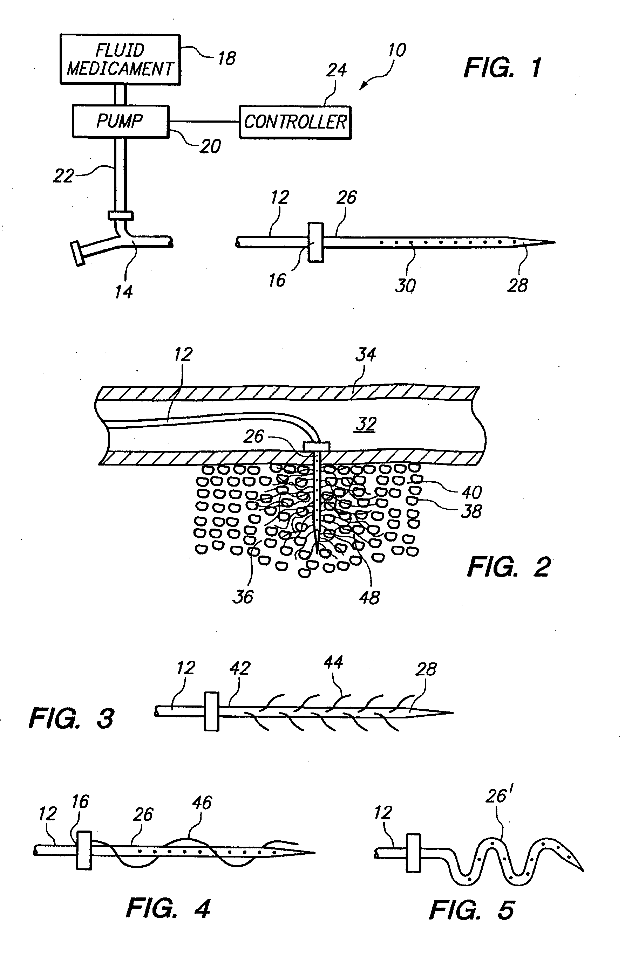

Referring initially to Fig. 1, a system for a transvenous soaker catheter in accordance with the present invention is shown and is generally designated 10. As shown, the system 10 includes an indwelling catheter 12 that has a proximal end 14 and a distal end 16. The system 10 also includes a source 18 of a fluid medicament (i.e. drug), and a pump 20 that can be used to pump the fluid medicament from the source 18 through a fluid line 22. In order to control the flow of this fluid medicament, the system 10 further includes a controller 24 that is electronically connected to the pump 20. In this combination, the controller 24 can be preprogrammed to operate the pump 20 according to a predetermined schedule. Specifically, this schedule can include the fluid flow rate of the fluid medicament being pumped by the pump 20, as well as the time duration of the fluid flow.

Still referring to Fig. 1, it will be seen that the fluid line 22 is connected in fluid communication with the proximal end 14 of the catheter 12. Thus, fluid medicament from the source 18 can be pumped directly to the catheter 12 in accordance with preprogrammed instructions from the controller 24. Fig. 1 also shows that the system 10 includes an infusion member 26 that is affixed to the distal end 16 of the catheter 12. For the purposes of the present invention, the infusion member 26 is preferably an elongated hollow needle 28 that is formed with a plurality of holes 30. As shown, the holes 30 are preferably aligned along the axis of the hollow needle 28. For its use in the system 10, the hollow needle 28 can be made of stainless steel and the holes 30 can be created using well known laser techniques.

Turning now to Fig. 2, the infusion member 26 is shown in its intended operational environment. Specifically, Fig. 2 shows the catheter 12 positioned in the lumen 32 of a vein 34. In this instance, the infusion member 26 of the system 10 has been embedded into the tissue 36 of an organ in the human body (not shown). Further, the tissue 36 in Fig. 2 is shown to anatomically comprise a plurality of cells 38 that are surrounded by extracellular fluid 40. As will be appreciated by the skilled artisan, although the discussion here considers the tissue 36 to be organ tissue, it is to be appreciated that other types of tissue (e.g. muscular tissue) may also be the intended target tissue 36 for therapeutic intervention with the system 10.

An alternative to the infusion member 26 (shown in Figs. 1 and 2) is an infusion member 42 as shown in Fig. 3. Like the infusion member 26, infusion member 42 incorporates an elongated hollow needle 28. Instead of holes 30, however, the infusion member 42 incorporates biodegradable fibers 44 that are positioned along the length of the needle 28. For use in the system 10, the biodegradable fibers 44 need to be permeable to the fluid medicament that is to be used and, thus, they are in fluid communication with the source 18.

Additional alternate embodiments of components for use with the system 10 are shown in Figs. 4 and 5. Specifically, Fig. 4 shows a helical shaped wire 46 that is affixed to the distal end 16 of the catheter 12. As intended for the system 10, this helical shaped wire 46 can be screwed into the target tissue 36 to provide additional stabilization for the infusion member 26 during operation of the system 10. Another alternative is shown in Fig. 5. There, an infusion member 26' is shown configured with a substantially helical shape. In this configuration, the infusion member 26' can, itself, be screwed into the target tissue 36 for additional stabilization.

In the operation of the system 10 of the present invention, a fluid medicament is selected for use and provided as the fluid source 18. Controller 24 is then programmed for the controlled operation of the pump 20. Specifically, a fluid flow regimen is established for the pump 20, with a prescribed fluid flow rate, and scheduled operational time durations (periodic or continuous). Then, with an infusion member 26 affixed to its distal end 16, the catheter 12 is advanced through the venous system of a patient until the infusion member 26 has been positioned adjacent target tissue 36 at the selected site. The infusion member 26 is then embedded into the target tissue 36 (see Fig. 2), by methods well known in the pertinent art. Lastly, the controller 24 is activated.

As indicated in Fig. 2, operation of the system 10 causes an infused medicament 48 to mix with extracellular fluid 40 in the target tissue 36. Importantly, the concentration of fluid medicament in source 18, as well as the rate, and the time duration of fluid flow provided by pump 20 are controllable. Consequently, the concentration gradient of infused medicament 48 in the target tissue 36 is also controllable.

While the particular Transvenous Soaker Catheter as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

## Claims

1. A system for infusing a fluid medicament into a volume of tissue which comprises:
a catheter having a proximal end and a distal end;
a source of fluid medicament connected in fluid communication with the proximal end of the catheter;
an infusion member affixed to the distal end of the catheter;
a means for embedding the infusion member into the volume of tissue to stabilize the infusion member in the volume of tissue; and
a pump means for transferring the fluid medicament from the source, through the catheter and through the infusion member at a predetermined fluid flow rate and for a predetermined time duration, for infusing fluid medicament into extracellular fluid in the volume of tissue.

2. A system as recited in claim 1 further comprising a plurality of biodegradable fibers affixed to the infusion member for transferring fluid medicament from the system into the extracellular fluid in the volume of tissue.

3. A system as recited in claim 1 wherein the infusion member is formed with at least one orifice for transferring fluid medicament from the system into the extracellular fluid in the volume of tissue.

4. A system as recited in claim 1 wherein the embedding means is a helical screw mounted on the distal end of the catheter.

5. A system as recited in claim 1 wherein the predetermined time duration is greater than 5 minutes.

6. A system as recited in claim 1 wherein the catheter is an indwelling catheter.

7. A system as recited in claim 1 wherein the pump means is adjustable to control the volumetric flow rate of fluid medicament through the catheter.

8. A system as recited in claim 1 wherein the pump means is a pacemaker.

9. A system as recited in claim 1 wherein the catheter is positioned in the venous system of a vasculature.

10. A system for infusing a fluid medicament which comprises:
a source of the fluid medicament;
a means for positioning a catheter in the vasculature of a patient, the catheter having a proximal end and a distal end, with an infusion member affixed to the distal end and the fluid medicament source attached in fluid communication with the proximal end thereof;
a means for embedding the infusion member into a volume of tissue;
a means for stabilizing the infusion member in the volume of tissue; and
a pumping means for transferring the fluid medicament from the source, through the catheter and through the infusion member for infusing fluid medicament at a predetermined fluid flow rate and for a predetermined time duration, for infusing fluid medicament into extracellular fluid in the volume of tissue.

11. A system as recited in claim 10 further comprising a plurality of biodegradable fibers affixed to the infusion member for transferring fluid medicament from the system into extracellular fluid in the volume of tissue.

12. A system as recited in claim 10 wherein the infusion member is formed with at least one orifice for transferring fluid medicament from the system into extracellular fluid in the volume of tissue.

13. A system as recited in claim 10 wherein the embedding means is a helical screw mounted on the distal end of the catheter.

14. A system as recited in claim 10 wherein the pumping means is adjustable to control the volumetric flow rate of fluid medicament through the catheter.

15. A system as recited in claim 10 wherein the catheter is positioned in the venous system of the vasculature.

16. A method for infusing a fluid medicament into a volume of tissue which comprises the steps of:
positioning a catheter in the vasculature of a patient, the catheter having a proximal end and a distal end, with an infusion member affixed to the distal end of the catheter, and with a source of the fluid medicament attached in fluid communication with the proximal end of the catheter;
embedding the infusion member into a volume of tissue; and
transferring the fluid medicament from the source, through the catheter and through the infusion member for infusing fluid medicament into extracellular fluid in the volume of tissue, at a predetermined fluid flow rate and for a predetermined time duration.
